# EUROPEAN PATENT APPLICATION

(11) **EP 3 385 711 A1**
(43) Date of publication of application: **10.10.2018**
(21) Application number: 17190719.9
(22) Date of filing: 12.09.2017
(51) Int. Cl.: G01N 33/50

(54) **URINE FLOW CYTOMETRY AS BIOMARKER OF RENAL DISEASES**

(30) Priority: 05.04.2017 EP 17165128
(71) Applicant: Charité - Universitätsmedizin Berlin, 10117 Berlin (DE)
(72) Inventor: The designation of the inventor has not yet been filed
(74) Representative: Schulz Junghans Patentanwälte PartGmbB

(57) **Abstract**

The invention provides a method of assigning to a patient a likelihood of having a kidney disease, or a likelihood of undergoing kidney transplant rejection, comprising the steps of providing a urine sample from the patient and determining the concentration of T cells, podocytes and proximal tubular epithelial cells. The ratios of these cell types are used for determining the risk of a kidney disease or transplant rejection.

## Description

The present invention relates to the detection of kidney diseases and kidney transplant rejection by analysis of the concentration of T cells, podocytes, tubular epithelial cells and their ratios in urine samples.

### Description

Kidney diseases are currently mostly diagnosed based on assessment of the glomerular filtration rate (creatinine clearance), analysis of the permeability of the blood-urine-barrier (proteinuria), and microscopic analysis of the cells present in the urine. The microscopic analysis is a semi-quantitative detection of usually unstained cells and highly depends on the examiner. The current non-invasive methods do not guarantee a high sensitivity and specificity.

Kidney transplantation is the best therapy for patients with terminal kidney insufficiency. Every transplantation however bears the risk of transplant rejection, recurrence of the underlying disease or chronic transplant damage. If impaired or deteriorating transplant function is detected in a patient by one of the above-mentioned methods, the patient usually has to undergo kidney biopsy in order to clarify the cause underlying the impaired kidney function. In the case of transplant rejection, acute cellular, acute humoral and chronic humoral transplant rejection can be distinguished. Depending on the cause underlying the impaired kidney function, a suitable therapy can be determined.

Based on the above-mentioned state of the art, the objective of the present invention is to provide a non-invasive method to determine the likelihood of a patient having a kidney disease or undergoing transplant rejection. This objective is attained by the claims of the present specification.

### Terms and definitions

The term "acute rejection" in the context of the present specification relates to a form of rejection that may begin as early as five days after surgery. The risk is highest in the first months after transplantation, but acute rejection can also occur years later.

The term "acute cellular rejection" in the context of the present specification relates to a form of acute rejection that is mainly mediated by T cells that invade the transplant.

The "term acute humoral rejection" in the context of the present specification relates to a form of acute rejection that is mainly mediated by antibodies that cross-react with the transplant.

The term "chronic rejection" in the context of the present specification relates to a form of rejection that develops more slowly than acute reaction. Chronic rejection may develop from recurring, treated episodes of acute rejection. Chronic rejection is generally considered poorly amenable to treatment.

The term "chronic humoral rejection" in the context of the present specification relates to a form of chronic rejection that and is mediated by antibodies that cross-react with the transplant.

The term "healthy subject" in the context of the present specification relates to a person that has not been diagnosed with a kidney disease.

The term "high dose cortisone" in the context of the present specification relates to a daily adult cortisone dose of at least 1 mg per kg body weight.

The term "increased immune therapy" in the context of the present specification relates to administration of additional immunosuppressive therapy, which usually consists of high dose cortisone and immune cell depleting antibodies or cytokine blocking antibodies.

The term "ligand specifically binding to a molecule" in the context of the present specification relates to antibodies and antibody-like molecules.

The term "antibody-like molecule" in the context of the present specification refers to a molecule capable of specific binding to another molecule or target with high affinity / a Kd ≤ 10E⁻⁸ mol/l. An antibody-like molecule binds to its target similarly to the specific binding of an antibody. The term antibody-like molecule encompasses a repeat protein, such as a designed ankyrin repeat protein (Molecular Partners, Zurich), a polypeptide derived from armadillo repeat proteins, a polypeptide derived from leucine-rich repeat proteins and a polypeptide derived from tetratricopeptide repeat proteins.

The term "antibody-like molecule" further encompasses a polypeptide derived from protein A domains, a polypeptide derived from fibronectin domain FN3, a polypeptide derived from consensus fibronectin domains, a polypeptide derived from lipocalins, a polypeptide derived from Zinc fingers, a polypeptide derived from Src homology domain 2 (SH2), a polypeptide derived from Src homology domain 3 (SH3), a polypeptide derived from PDZ domains, a polypeptide derived from gamma-crystallin, a polypeptide derived from ubiquitin, a polypeptide derived from a cysteine knot polypeptide, a polypeptide derived from knottin, a nucleic acid aptamer and a peptide aptamer.

The term "specifically binding" in the context of the present specification relates binding of one molecule to another molecule with a Kd ≤ 10E⁻⁸ mol/l.

### Brief description of the figures

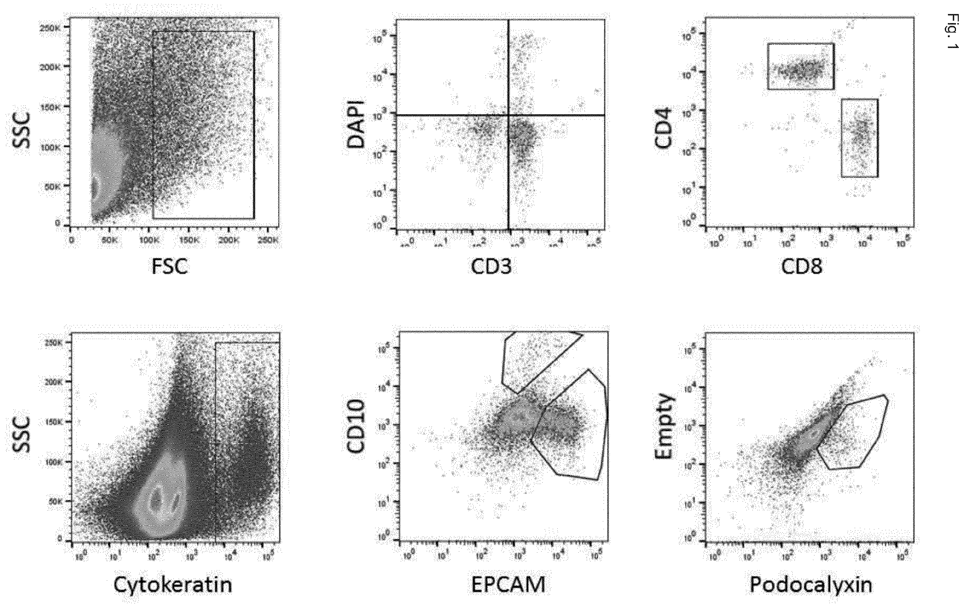

Fig. 1 shows the gating strategy for cell type quantification. Upper panel (from left to right): lymphocyte gate, DAPI⁻CD3⁺ gate, CD4⁺ T cell gate and CD8⁺ T cell gate. Lower panel (from left to right): cytokeratin⁺ gate, EPCAM⁺ tubular epithelial cell gate and CD10⁺ tubular epithelial cell gate, podocyte gate.

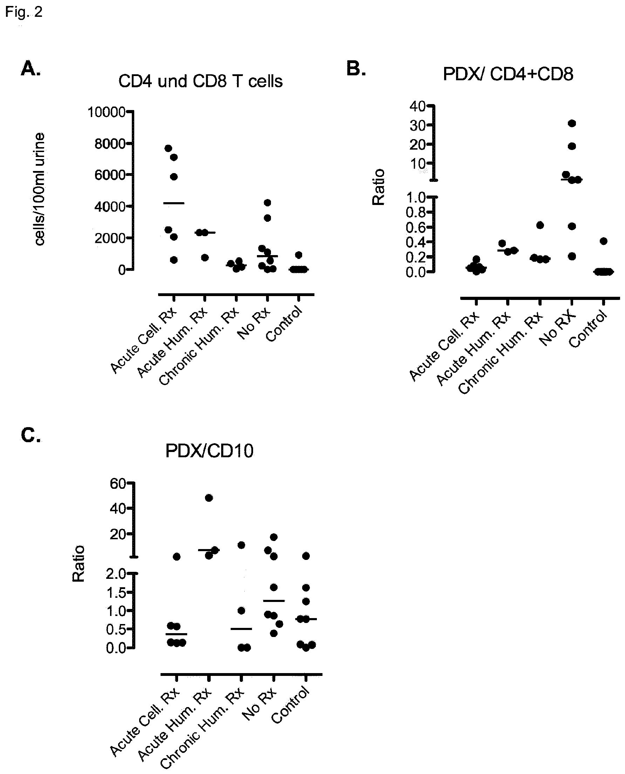

Fig. 2 shows the concentration of T cells (CD4⁺ T cells and CD8⁺ T cells) (A), the ratio of podocytes to T cells (B) and the ratio of podocytes to CD10⁺ proximal tubular epithelial cells(C) in urine samples obtained from kidney transplant patients. The groups acute cellular rejection, acute humoral rejection, chronic humoral rejection and no rejection have been determined by kidney biopsy. The control group are patients with stable kidney function after transplantation.

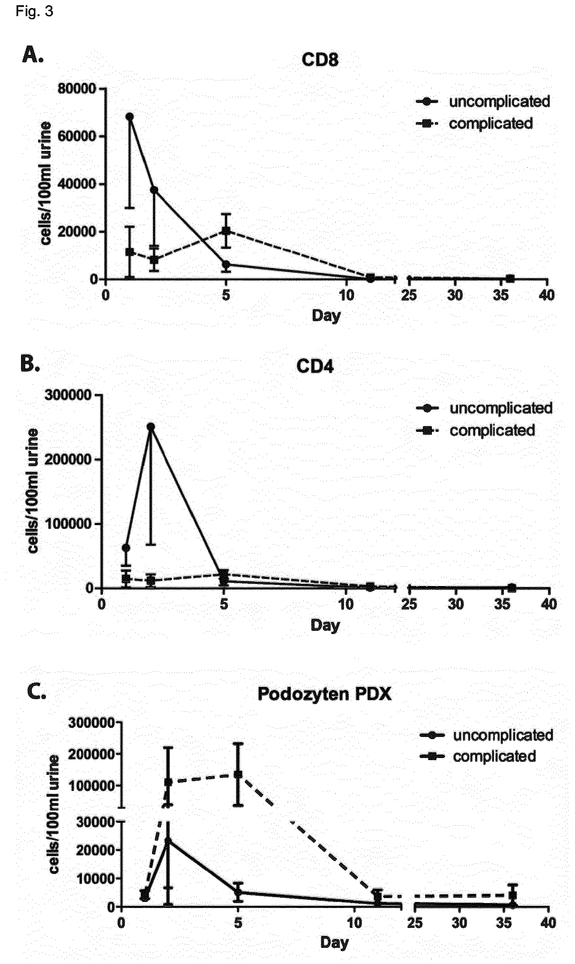

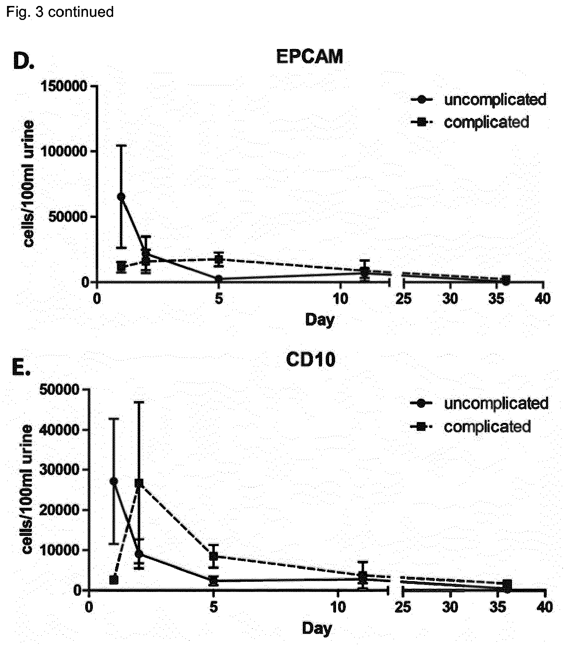

Fig. 3 shows the concentrations of CD4⁺ T cells (A), CD8⁺ T cells (B), podocytes (C), distal tubular epithelial cells (D) and proximal tubular epithelial cells (E) in urine samples obtained from kidney transplant patients after recent transplantation. The X-axis indicates the day after surgery, with day 0 being the kidney transplantation.

According to a first aspect of the invention, a method of assigning to patient a likelihood of having a kidney disease or a likelihood of undergoing kidney transplant rejection is provided. The method comprises the steps of
a. providing a urine sample from the patient;
b. determining a concentration of T cells, a concentration of podocytes and a concentration of proximal tubular epithelial cells, and optionally a concentration of distal tubular epithelial cells in the urine sample.

The skilled person is aware that following step a and previous to step b, the cells are isolated from the urine sample.

In certain embodiments, the T cells are characterized by expression of CD3 and CD4. In certain embodiments, the T cells are characterized by expression of CD3 and CD8. In certain embodiments, the T cells are characterized by expression of CD3, CD4 and CD8. In certain embodiments, the T cells are characterized by expression of CD3. In certain embodiments, the T cells are characterized by expression of CD4 and CD8.

In certain embodiments, the podocytes are characterized by expression of podocalyxin. In certain embodiments, the podocytes are characterized by expression of podocin. In certain embodiments, the podocytes are characterized by expression of nephrin. In certain embodiments, the podocytes are characterized by expression of WT1 (Wilms-Tumor-Protein 1).

In certain embodiments, the proximal tubular epithelial cells are characterized by expression of CD10. In certain embodiments, the proximal tubular epithelial cells are characterized by expression of CD10 and cytokeratin. In certain embodiments, the distal tubular epithelial cells are characterized by expression of EPCAM. In certain embodiments, the distal tubular epithelial cells are characterized by expression of EPCAM and cytokeratin.

Step b is effected by contacting the cells isolated from the urine sample with a set of detectable ligands. The set comprises members capable of binding to each of CD4, CD8, CD10, at least one of podocalyxin, podocin, nephrin and WT1, and optionally cytokeratin, CD3 and/or EPCAM. Each of said members is capable of specifically binding to one of CD3, CD4, CD8, podocalyxin, podocin, nephrin, WT1, CD10, cytokeratin and EPCAM.

In the context of the present specification, CD4 refers to a human protein having the UniProt ID P01730.

In the context of the present specification, CD8 refers to a human protein having the UniProt ID P01732.

In the context of the present specification, podocin refers to a human protein having the UniProt ID Q9NP85.

In the context of the present specification, WT1 refers to a human protein having the UniProt ID P19544.

In the context of the present specification, nephrin refers to a human protein having the UniProt ID 060500.

In the context of the present specification, podocalyxin refers to a human protein having the UniProt ID 000592.

In certain embodiments, the ligand is an antibody.

In certain embodiments, the concentration of T cells, the concentration of podocytes, the concentration of proximal tubular epithelial cells and optionally, the concentration of distal tubular epithelial cells are each determined by flow cytometry.

In certain embodiments, the kidney disease is selected from the group comprising (kidney) transplant rejection, acute renal failure, acute renal failure due to autoimmune disease, lupus nephritis, ANCA associated glomerulonephritis and diabetic kidney disease.

In certain embodiments, the kidney disease is transplant rejection.

In certain embodiments, the kidney disease is acute cellular transplant rejection.

In certain embodiments, the kidney disease is acute humoral transplant rejection.

In certain embodiments, the kidney disease is chronic humoral transplant rejection.

In certain embodiments, the kidney disease is acute cellular transplant rejection or acute humoral transplant rejection.

In certain embodiments, the patient showed signs of impaired kidney function prior to collection of the urine sample. In certain embodiments, the patient showed proteinuria or reduced creatinine clearance / increased creatinine levels.

In certain embodiments, the patient showed signs of impaired transplant function prior to collection of the urine sample. In certain embodiments, the patient showed proteinuria or reduced creatinine clearance / increased creatinine levels.

Patients showing signs of impaired kidney (or kidney transplant) function are often assigned to kidney biopsy for clarification of the cause underlying the impaired kidney function. The method of the invention allows to reduce the number of unnecessary kidney biopsies and facilitates the early identification of patients who should receive a kidney biopsy.

In certain embodiments, the method further comprises the steps of
c. determining a podocyte/T cell ratio and a podocyte/proximal tubular epithelial cell ratio from the concentration of cells determined in step b;
d. comparing the podocyte/T cell ratio and the podocyte/proximal tubular epithelial cell ratio determined in step c with a respective threshold;
e. assigning to the patient a high likelihood of having a kidney disease or of undergoing kidney transplant rejection
   i. if the podocyte/T cell ratio is below a podocyte/T cell threshold or
   ii. if the podocyte/proximal tubular epithelial cell ratio is above a podocyte/proximal tubular epithelial cell threshold.

In certain embodiments, the method comprises the steps of
c. determining a podocyte/T cell ratio and a podocyte/proximal tubular epithelial cell ratio from the concentration of cells determined in step b;
d. comparing the concentration of T cells determined in step b and the podocyte/T cell ratio and the podocyte/proximal tubular epithelial cell ratio determined in step c with a respective threshold;
e. assigning to the patient a high likelihood of transplant rejection
   i. if the concentration of T cells is above a T cell threshold and the podocyte/T cell ratio is below a podocyte/T cell threshold or
   ii. if the concentration of T cells is above the T cell threshold and the podocyte/proximal tubular epithelial cell ratio is above a podocyte/proximal tubular epithelial cell threshold.

In certain embodiments, the method comprises the steps of
c. determining a podocyte/T cell ratio and a podocyte/proximal tubular epithelial cell ratio from the concentration of cells determined in step b;
d. comparing the concentration of T cells determined in step b and the podocyte/T cell ratio and the podocyte/proximal tubular epithelial cell ratio determined in step c with a respective threshold;
e. assigning to the patient a high likelihood of transplant rejection
   i. if the concentration of T cells is above a first T cell threshold and the podocyte/T cell ratio is below a podocyte/T cell threshold or
   ii. if the concentration of T cells is above a second T cell threshold and the podocyte/proximal tubular epithelial cell ratio is above a podocyte/proximal tubular epithelial cell threshold
wherein the first T cell threshold is higher than the second T cell threshold.

In certain embodiments, the parameters of the above-mentioned clauses (i) are indicative for an acute cellular rejection.

In certain embodiments, the parameters of the above-mentioned clauses (ii) are indicative for an acute humoral rejection.

In certain embodiments, the T cell threshold is above the average T cell concentration in healthy subjects. In certain embodiments, the T cell threshold is at least 10 x above the average T cell concentration in healthy subjects. In certain embodiments, the T cell threshold is at least 50 x above the average T cell concentration in healthy subjects. In certain embodiments, the T cell threshold is at least 100 x above the average T cell concentration in healthy subjects.

In certain embodiments, the kidney disease is transplant rejection and the T cell threshold is above the average T cell concentration in patients showing no complications after kidney transplantation. In certain embodiments, the kidney disease is transplant rejection and the T cell threshold is at least 2 x above the average T cell concentration in patients showing no complications after kidney transplantation. In certain embodiments, the kidney disease is transplant rejection and the T cell threshold is at least 3 x above the average T cell concentration in patients showing no complications after kidney transplantation. In certain embodiments, the kidney disease is transplant rejection and the T cell threshold is at least 5 x above the average T cell concentration in patients showing no complications after kidney transplantation.

The skilled person is aware that in instances where a threshold is determined with reference to an average concentration or ratio in patients showing no complications after kidney transplantation, this relates to the average concentration or ratio determined at time point corresponding to the time point at which the sample from the patient is obtained.

In certain embodiments, the T cell threshold is 2000 - 500 T cells/100 ml urine. In certain embodiments, the T cell threshold is 1500 - 550 T cells/100 ml urine. In certain embodiments, the T cell threshold is 1000 - 600 T cells/100 ml urine. In certain embodiments, the T cell threshold is approximately 600 T cells/100 ml urine.

In certain embodiments, the kidney disease is transplant rejection and the T cell threshold is 2000 - 500 T cells/100 ml urine. In certain embodiments, the kidney disease is transplant rejection and the T cell threshold is 1500 - 550 T cells/100 ml urine. In certain embodiments, the kidney disease is transplant rejection and the T cell threshold is 1000 - 600 T cells/100 ml urine. In certain embodiments, the kidney disease is transplant rejection and the T cell threshold is approximately 600 T cells/100 ml urine.

In certain embodiments, the kidney disease is transplant rejection and the podocyte/T cell threshold is above the average the podocyte/T cell ratio in patients showing no complications after kidney transplantation. In certain embodiments, the kidney disease is transplant rejection and the podocyte/T cell threshold is not above (≤) 5 x the average the podocyte/T cell ratio in patients showing no complications after kidney transplantation.

In certain embodiments, the podocyte/T cell threshold is 0.01 - 0.3. In certain embodiments, the podocyte/T cell threshold is 0.02 - 0.2. In certain embodiments, the podocyte/T cell threshold is 0.1 - 0.2. In certain embodiments, the podocyte/T cell threshold is approximately 0.1. In certain embodiments, the podocyte/T cell threshold is approximately 0.2.

In certain embodiments, the kidney disease is transplant rejection and the podocyte/T cell threshold is 0.01 - 0.3. In certain embodiments, the kidney disease is transplant rejection and the podocyte/T cell threshold is 0.02 - 0.2. In certain embodiments, the kidney disease is transplant rejection and the podocyte/T cell threshold is 0.1 - 0.2. In certain embodiments, the kidney disease is transplant rejection and the podocyte/T cell threshold is approximately 0.1. In certain embodiments, the kidney disease is transplant rejection and the podocyte/T cell threshold is approximately 0.2.

In certain embodiments, the kidney disease is transplant rejection and the podocyte/ proximal tubular epithelial cell threshold is above the average the podocyte/proximal tubular epithelial cell threshold in patients showing no complications after kidney transplantation. In certain embodiments, the kidney disease is transplant rejection and the podocyte/proximal tubular epithelial cell threshold is at least 2 x above the average the podocyte/proximal tubular epithelial cell ratio in patients showing no complications after kidney transplantation. In certain embodiments, the kidney disease is transplant rejection and the podocyte/proximal tubular epithelial cell threshold is at least 3 x above the average the podocyte/proximal tubular epithelial cell ratio in patients showing no complications after kidney transplantation. In certain embodiments, the kidney disease is transplant rejection and the podocyte/proximal tubular epithelial cell threshold is at least 5 x above the average the podocyte/proximal tubular epithelial cell ratio in patients showing no complications after kidney transplantation.

In certain embodiments, the podocyte/proximal tubular epithelial cell threshold is 1 - 13. In certain embodiments, the podocyte/proximal tubular epithelial cell threshold is 1.5 to 10. In certain embodiments, the podocyte/proximal tubular epithelial cell threshold is 2 - 5. In certain embodiments, the podocyte/proximal tubular epithelial cell threshold is approximately 3.

In certain embodiments, the kidney disease is transplant rejection and the podocyte/proximal tubular epithelial cell threshold is 1 - 13. In certain embodiments, the kidney disease is transplant rejection and the podocyte/proximal tubular epithelial cell threshold is 1.5 to 10. In certain embodiments, the kidney disease is transplant rejection and the podocyte/proximal tubular epithelial cell threshold is 2 - 5. In certain embodiments, the kidney disease is transplant rejection and the podocyte/proximal tubular epithelial cell threshold is approximately 3.

In certain embodiments, a high likelihood of *acute* cellular transplant rejection is assigned to the patient if the concentration of T cells is above the T cell threshold and the podocyte/T cell ratio is below the podocyte/T cell threshold.

In certain embodiments, a high likelihood of *acute humoral* transplant rejection is assigned to the patient if the concentration of T cells is above the T cell threshold and the podocyte/proximal tubular epithelial cell ratio is above the podocyte/proximal tubular epithelial cell threshold.

In certain embodiments, a high likelihood of *acute* cellular transplant rejection is assigned to the patient if the podocyte/T cell ratio is below the podocyte/T cell threshold.

In certain embodiments, a high likelihood of *acute humoral* transplant rejection is assigned to the patient if the podocyte/proximal tubular epithelial cell ratio is above the podocyte/proximal tubular epithelial cell threshold.

In certain embodiments, a high likelihood of *acute* cellular transplant rejection is assigned to the patient if the concentration of T cells is above a first T cell threshold and the podocyte/T cell ratio is below a podocyte/T cell threshold. In certain embodiments, a high likelihood of *acute humoral* transplant rejection is assigned to the patient if the concentration of T cells is above a second T cell threshold and the podocyte/proximal tubular epithelial cell ratio is above the podocyte/proximal tubular epithelial cell threshold. In instances where a first and a second T cell threshold are used, the first T cell threshold is higher than the second T cell threshold

Discrimination between the different forms of transplant rejection is important for the resulting therapy of the patient. Therapies suitable for acute cellular transplant rejection include high dose cortisone, immune suppression therapy with increased doses of immune suppression drugs, therapeutic antibodies against T cells, anti-thymocyte globulin, Thymoglogulin, Atgam and other therapeutic antibodies including anti-TNF. Therapies suitable for acute humoral transplant rejection include high dose cortisone, plasma exchange, therapeutic antibodies against B cells, including Rituximab.

In certain embodiments, the concentration of T cells, the concentration of podocytes, the concentration of proximal tubular epithelial cells and/or the concentration of distal tubular epithelial cells determined in step b are each compared with a respective predetermined cell type threshold and a high likelihood of having a kidney disease or of undergoing kidney transplant rejection is assigned to the patient if at least two, particularly at least three, more particularly at least four, even more particularly all of said concentrations are above said respective predetermined thresholds. In certain embodiments, instead of the total CD4+ CD8+ T cell concentration, the concentration of CD4+ T cells and the concentration of CD8+ T cells is determined and compared with a predetermined CD4+ T cell threshold or a predetermined CD8+ T cell threshold, respectively.

In certain embodiments, a high likelihood of having a kidney disease is assigned to the patient if at least two of the concentrations are above the respective cell type thresholds. In certain embodiments, a high likelihood of having a kidney disease is assigned to the patient if at least three of the concentrations are above the respective cell type thresholds. In certain embodiments, a high likelihood of having a kidney disease is assigned to the patient if at least four of the concentrations are above the respective cell type thresholds. In certain embodiments, a high likelihood of having a kidney disease is assigned to the patient if all of the concentrations are above the respective cell type thresholds.

In certain embodiments, a high likelihood of having a kidney disease is assigned to the patient if the T cell concentration, in particular the concentration of CD8+ T cells is above the respective cell type threshold (T cell threshold or CD8+ T cell threshold, respectively), the podocyte concentration is above the podocyte threshold, the proximal tubular epithelial cell concentration is above the proximal tubular epithelial cell threshold and the distal tubular epithelial cell concentration is above the distal tubular epithelial cell threshold.

In certain embodiments, a high likelihood of having a kidney disease is assigned to the patient if the T cell concentration, in particular the concentration of CD8+ T cells is above the respective cell type threshold (T cell threshold or CD8+ T cell threshold, respectively), the podocyte concentration is above the podocyte threshold and the proximal tubular epithelial cell concentration is above the proximal tubular epithelial cell threshold.

In certain embodiments, a high likelihood of having a kidney disease is assigned to the patient if the T cell concentration, in particular the concentration of CD8+ T cells is above the respective cell type threshold (T cell threshold or CD8+ T cell threshold, respectively), the podocyte concentration is above the podocyte threshold, and the distal tubular epithelial cell concentration is above the distal tubular epithelial cell threshold.

In certain embodiments, a high likelihood of having a kidney disease is assigned to the patient if the T cell concentration, in particular the concentration of CD8+ T cells is above the respective cell type threshold (T cell threshold or CD8+ T cell threshold, respectively) and the podocyte concentration is above the podocyte threshold.

In certain embodiments, a high likelihood of having a kidney disease is assigned to the patient if the podocyte concentration is above the podocyte threshold, the proximal tubular epithelial cell concentration is above the proximal tubular epithelial cell threshold and the distal tubular epithelial cell concentration is above the distal tubular epithelial cell threshold.

In certain embodiments, a high likelihood of having a kidney disease is assigned to the patient if the podocyte concentration is above the podocyte threshold and the proximal tubular epithelial cell concentration is above the proximal tubular epithelial cell threshold.

In certain embodiments, a high likelihood of having a kidney disease is assigned to the patient if the podocyte concentration is above the podocyte threshold and the distal tubular epithelial cell concentration is above the distal tubular epithelial cell threshold.

In certain embodiments, a high likelihood of having a kidney disease is assigned to the patient if the total concentration of CD8+ T cells, podocytes, proximal tubular epithelial cells and distal tubular epithelial cells is above a collective cell concentration threshold.

In certain embodiments, a high likelihood of transplant rejection is assigned to the patient if at least two of the concentrations are above the respective cell type thresholds. In certain embodiments, a high likelihood of transplant rejection is assigned to the patient if at least three of the concentrations are above the respective cell type thresholds. In certain embodiments, a high likelihood of transplant rejection is assigned to the patient if at least four of the concentrations are above the respective cell type thresholds. In certain embodiments, a high likelihood of transplant rejection is assigned to the patient if all of the concentrations are above the respective cell type thresholds.

In certain embodiments, a high likelihood of transplant rejection is assigned to the patient if the T cell concentration, in particular the concentration of CD8+ T cells is above the respective cell type threshold (T cell threshold or CD8+ T cell threshold, respectively), the podocyte concentration is above the podocyte threshold, the proximal tubular epithelial cell concentration is above the proximal tubular epithelial cell threshold and the distal tubular epithelial cell concentration is above the distal tubular epithelial cell threshold.

In certain embodiments, a high likelihood of transplant rejection is assigned to the patient if the T cell concentration, in particular the concentration of CD8+ T cells is above the respective cell type threshold (T cell threshold or CD8+ T cell threshold, respectively), the podocyte concentration is above the podocyte threshold and the proximal tubular epithelial cell concentration is above the proximal tubular epithelial cell threshold.

In certain embodiments, a high likelihood of transplant rejection is assigned to the patient if the T cell concentration, in particular the concentration of CD8+ T cells is above the respective cell type threshold (T cell threshold or CD8+ T cell threshold, respectively), the podocyte concentration is above the podocyte threshold, and the distal tubular epithelial cell concentration is above the distal tubular epithelial cell threshold.

In certain embodiments, a high likelihood of transplant rejection is assigned to the patient if the T cell concentration, in particular the concentration of CD8+ T cells is above the respective cell type threshold (T cell threshold or CD8+ T cell threshold, respectively) and the podocyte concentration is above the podocyte threshold.

In certain embodiments, a high likelihood of transplant rejection is assigned to the patient if the podocyte concentration is above the podocyte threshold, the proximal tubular epithelial cell concentration is above the proximal tubular epithelial cell threshold and the distal tubular epithelial cell concentration is above the distal tubular epithelial cell threshold.

In certain embodiments, a high likelihood of transplant rejection is assigned to the patient if the podocyte concentration is above the podocyte threshold and the proximal tubular epithelial cell concentration is above the proximal tubular epithelial cell threshold.

In certain embodiments, a high likelihood of transplant rejection is assigned to the patient if the podocyte concentration is above the podocyte threshold and the distal tubular epithelial cell concentration is above the distal tubular epithelial cell threshold.

In certain embodiments, a total concentration of CD8+ T cells, CD4+ cells, podocytes, proximal tubular epithelial cells and distal tubular epithelial cells is determined and a high likelihood of having a kidney disease or of undergoing kidney transplant rejection is assigned to the patient if the total concentration is above a collective cell concentration threshold.

In certain embodiments, the T cell threshold is above the average T cell concentration in healthy subjects. In certain embodiments, the T cell threshold is at least 100 x above the average T cell concentration in healthy subjects.

In certain embodiments, the kidney disease is transplant rejection and the T cell threshold is above the average T cell concentration in patients showing no complications after kidney transplantation. In certain embodiments, the kidney disease is transplant rejection and the T cell threshold is at least 3 x above the average T cell concentration in patients showing no complications after kidney transplantation.

In certain embodiments, the CD4+ T cell threshold is above the average CD4+ T cell concentration in healthy subjects. In certain embodiments, the CD4+ T cell threshold is at least 50 x, above the average CD4+ T cell concentration in healthy subjects.

In certain embodiments, the kidney disease is transplant rejection and the CD4+ T cell threshold is above the average CD4+ T cell concentration in patients showing no complications after kidney transplantation. In certain embodiments, the kidney disease is transplant rejection and the CD4+ T cell threshold is at least 3 x above the average CD4+ T cell concentration in patients showing no complications after kidney transplantation.

In certain embodiments, the CD8+ T cell threshold is above the average CD8+ T cell concentration in healthy subjects. In certain embodiments, the CD8+ T cell threshold is at least 50 x above the average CD8+ T cell concentration in healthy subjects.

In certain embodiments, the kidney disease is transplant rejection and the CD8+ T cell threshold is above the average CD8+ T cell concentration in patients showing no complications after kidney transplantation. In certain embodiments, the kidney disease is transplant rejection and the CD8+ T cell threshold is at least 3 x above the average CD8+ T cell concentration in patients showing no complications after kidney transplantation.

In certain embodiments, the podocyte threshold is above the average podocyte concentration in healthy subjects. In certain embodiments, the podocyte threshold is at least 5 x above the average podocyte concentration in healthy subjects.

In certain embodiments, the kidney disease is transplant rejection and the podocyte threshold is above the average podocyte concentration in patients showing no complications after kidney transplantation. In certain embodiments, the kidney disease is transplant rejection and the podocyte threshold is at least 5 x above the average podocyte concentration in patients showing no complications after kidney transplantation.

In certain embodiments, the proximal tubular epithelial cell threshold is above the average proximal tubular epithelial cell concentration in healthy subjects. In certain embodiments, the proximal tubular epithelial cell threshold is at least 5 x above the average proximal tubular epithelial cell concentration in healthy subjects.

In certain embodiments, the kidney disease is transplant rejection and the proximal tubular epithelial cell is above the average proximal tubular epithelial cell concentration in patients showing no complications after kidney transplantation. In certain embodiments, the kidney disease is transplant rejection and the proximal tubular epithelial cell threshold is at least 5x above the average proximal tubular epithelial cell concentration in patients showing no complications after kidney transplantation.

In certain embodiments, the distal tubular epithelial cell threshold is above the average distal tubular epithelial cell concentration in healthy subjects. In certain embodiments, the distal tubular epithelial cell threshold is at least 5x above the average distal tubular epithelial cell concentration in healthy subjects.

In certain embodiments, the kidney disease is transplant rejection and the distal tubular epithelial cell is above the average distal tubular epithelial cell concentration in patients showing no complications after kidney transplantation. In certain embodiments, the kidney disease is transplant rejection and the distal tubular epithelial cell threshold is at least 5 x above the average distal tubular epithelial cell concentration in patients showing no complications after kidney transplantation.

In certain embodiments, the collective cell concentration threshold is above the average total concentration of CD8+ T cells, podocytes, proximal tubular epithelial cells and distal tubular epithelial cells in healthy subjects. In certain embodiments, the collective cell concentration threshold is at least 5 x the average total concentration of CD8+ T cells, podocytes, proximal tubular epithelial cells and distal tubular epithelial cells in healthy subjects.

In certain embodiments, the collective cell concentration threshold is above the average total concentration of CD8+ T cells, podocytes, proximal tubular epithelial cells and distal tubular epithelial cells in healthy subjects. In certain embodiments, the collective cell concentration threshold is at least 5 x the average total concentration of CD8+ T cells, podocytes, proximal tubular epithelial cells and distal tubular epithelial cells in healthy subjects.

In certain embodiments, the collective cell concentration threshold is above the average total concentration of CD8+ T cells, podocytes, proximal tubular epithelial cells and distal tubular epithelial cells in patients showing no complications after kidney transplantation. In certain embodiments, the collective cell concentration threshold is at least 5 x the average total concentration of CD8+ T cells, podocytes, proximal tubular epithelial cells and distal tubular epithelial cells in patients showing no complications after kidney transplantation.

In certain embodiments, the collective cell concentration threshold is 20.000 - 200.000 cells/100 ml urine. In certain embodiments, the collective cell concentration threshold is 22.000 - 100.000 cells/100 ml urine. In certain embodiments, the collective cell concentration threshold is 23.000 - 50.000 cells/100 ml urine. In certain embodiments, the collective cell concentration threshold is 24.000 - 30.000 cells/100 ml urine. In certain embodiments, the collective cell concentration threshold is approximately 45.000-cells/100 ml urine.

In certain embodiments, the disease is transplant rejection and the collective cell concentration threshold is 20.000 - 200.000 cells/100 ml urine. In certain embodiments, the disease is transplant rejection and the collective cell concentration threshold is 22.000 - 100.000 cells/100 ml urine. In certain embodiments, the disease is transplant rejection and the collective cell concentration threshold is 23.000 - 50.000 cells/100 ml urine. In certain embodiments, the disease is transplant rejection and the collective cell concentration threshold is 24.000 - 30.000 cells/100 ml urine. In certain embodiments, the disease is transplant rejection and the collective cell concentration threshold is approximately 45.000 cells/100 ml urine.

In certain embodiments, the disease is transplant rejection and the collective cell concentration threshold on day 5 after transplantation is 20.000 - 200.000 cells/100 ml urine. In certain embodiments, the disease is transplant rejection and the collective cell concentration threshold on day 5 after transplantation is 22.000 - 100.000 cells/100 ml urine. In certain embodiments, the disease is transplant rejection and the collective cell concentration threshold on day 5 after transplantation is 23.000 - 50.000 cells/100 ml urine. In certain embodiments, the disease is transplant rejection and the collective cell concentration threshold on day 5 after transplantation is 24.000 - 30.000 cells/100 ml urine. In certain embodiments, the disease is transplant rejection and the collective cell concentration threshold on day 5 after transplantation is approximately 45.000-cells/100 ml urine.

The method of the invention allows identifying patients with a high likelihood of transplant rejection. Patients showing a high likelihood of transplant rejection according to the method of the invention should be assigned to special examination. If indicated by the result of this examination, patients showing cell concentrations above the respective thresholds should be assigned to kidney biopsy. The method of the invention allows reducing the number of unnecessary kidney biopsies and facilitates the early identification of patients who should receive a kidney biopsy.

In certain embodiments, the sample is provided on day 4 after transplantation or later. In certain embodiments, the sample is provided on day 5 after transplantation or later. In certain embodiments, the sample is provided on day 5 to several years after transplantation. In certain embodiments, the sample is provided on day 5 to 10 after transplantation. In certain embodiments, the sample is provided on day 5 to 7 after transplantation. In certain embodiments, the sample is provided on day 5.

In certain embodiments, two (or more) samples are provided, wherein
- a first sample is provided before day 5 after transplantation, and a second sample is provided after day 5 after transplantation, and
- a high likelihood of transplant rejection is assigned to the patient if
   i. in the first sample at least two, particularly at least three, more particularly at least four, even more particularly all of said concentrations are below a first respective cell type threshold and
   ii. in the second sample at least two, particularly at least three, more particularly at least four, even more particularly all of said concentrations are *above* a second respective cell type threshold.

In certain embodiments, in instances where only a subset of the five relevant cell types (CD8+ T cells, CD4+ T cells, podocytes, proximal tubular epithelial cells, distal tubular epithelial cells) is compared, the same cell types are compared in the first and second sample.

In certain embodiments, a high likelihood of transplant rejection is assigned to the patient if in the first sample the T cell concentration, in particular the CD4+ T cell concentration, is below a predetermined T cell threshold and in the in the second sample at least two, particularly at least three, more particularly at least four, even more particularly all of said concentrations are *above* a second respective cell type threshold.

In certain embodiments, a high likelihood of transplant rejection is assigned to the patient if in the first sample the T cell concentration, in particular the CD4+ T cell concentration, is below a predetermined T cell threshold and in the in the second sample the total concentration of CD8+ T cells, podocytes, proximal tubular epithelial cells and distal tubular epithelial cell is above a collective cell concentration threshold.

In certain embodiments, two (or more) samples are provided, wherein
- a first sample is provided before day 5 after transplantation, and a second sample is provided after day 5 after transplantation, and
- a high likelihood of transplant rejection is assigned to the patient if
   i. in the first sample the total concentration of CD8+ T cells, podocytes, proximal tubular epithelial cells and distal tubular epithelial cell is above a collective cell concentration threshold d and
   ii. in the second sample if the total concentration of CD8+ T cells, podocytes, proximal tubular epithelial cells and distal tubular epithelial cell is above a collective cell concentration threshold.

Alternatively, the first aspect of the invention may be described as a method of assigning to a kidney transplant patient a likelihood of currently undergoing transplant rejection.

Alternatively, the first aspect of the invention may be described as a method of assigning to a kidney transplant patient a likelihood of being at risk of transplant rejection. In this alternative description of the first aspect of the invention, urine samples may also be taken before surgery in order to predict a likelihood of rejection after transplantation.

According to a second aspect of the invention, a method of assigning a kidney transplant patient to increased immune suppression therapy is provided. The method comprises the steps of
- determining a likelihood of transplant rejection in said patient according to the method of any one of the above claims;
- assigning said patient to increased immune suppression therapy if a high likelihood of transplant rejection is determined.

In certain embodiments of this aspect of the invention, the patient is assigned to therapy suitable for acute cellular transplant rejection if a high likelihood of acute cellular transplant rejection is assigned to the patient according to the respective embodiment of the first aspect of the invention. In certain embodiments of this aspect of the invention, the patient is assigned to a therapy selected from the group comprising treatment with high dose cortisone, immune suppression therapy with increased doses of immune suppression drugs, treatment with therapeutic antibodies against T cells, treatment with an anti-thymocyte globulin and treatment with other therapeutic antibodies including anti-TNF.

In certain embodiments of this aspect of the invention, the patient is assigned to therapy suitable for acute humoral transplant rejection if a high likelihood of acute humoral transplant rejection is assigned to the patient according to the respective embodiment of the first aspect of the invention. In certain embodiments of this aspect of the invention, the patient is assigned to a therapy selected from the group comprising treatment with high dose cortisone, plasma exchange and treatment with therapeutic antibodies against B cells, including Rituximab.

According to another aspect of the invention, a method of assigning a kidney transplant patient to a kidney biopsy is provided. The method comprises the steps of
- determining a likelihood of transplant rejection in said patient according to the method of any one of the above claims;
- assigning said patient to kidney biopsy if a high likelihood of transplant rejection is determined.

According to another aspect of the invention, a kit of parts for determining the likelihood of a patient having a kidney disease or undergoing kidney transplant rejection is provided. In other words, a kit for diagnosing a kidney disease or transplant rejection is provided. The kit comprises an anti-CD8 and/or an anti-CD4 antibody, an anti-CD10 antibody, an antibody selected from an anti-podocalyxin antibody, an anti- podocin antibody, an anti-nephrin antibody and an anti-WT1 antibody, and optionally an anti-EPCAM antibody and/or an anti-cytokeratin antibody. The antibodies are suitable for fluorescence based flow cytometry.

In certain embodiments of this aspect of the invention, the kit comprises an anti-CD8 and/or an anti-CD4 antibody, an anti-podocalyxin antibody and an anti-CD10 antibody. In certain embodiments of this aspect of the invention, the kit comprises an anti-CD8 antibody, an anti-CD4 antibody, an anti-podocalyxin antibody and an anti-CD10 antibody. In certain embodiments of this aspect of the invention, the kit comprises an anti-CD8 and/or an anti-CD4 antibody, an anti-podocalyxin antibody, an anti-CD10 antibody and an anti-EPCAM and/or an anti-cytokeratin antibody. In certain embodiments of this aspect of the invention, the kit comprises an anti-CD8 antibody, an anti-CD4 antibody, an anti-podocalyxin antibody, an anti-CD10 antibody and an anti-EPCAM and/or an anti-cytokeratin antibody. In certain embodiments of this aspect of the invention, the kit comprises an anti-CD8 antibody, an anti-CD4 antibody, an anti-podocalyxin antibody, an anti-CD10 antibody, an anti-EPCAM and an anti-cytokeratin antibody.

According to yet another aspect of the invention, a use of a combination of antibodies for determining the likelihood of a patient having a kidney disease or undergoing kidney transplant rejection is provided. The combination of antibodies comprises an anti-CD8 and/or an anti-CD4 antibody, an anti-CD10 antibody, an antibody selected from an anti-podocalyxin antibody, an anti- podocin antibody, an anti-nephrin antibody and an anti-WT1 antibody, and optionally an anti-EPCAM antibody and/or an anti-cytokeratin antibody.

According to yet another aspect of the invention, a system for determining the likelihood of a patient having a kidney disease or undergoing kidney transplant rejection is provided. The system comprises a device for determining the frequency of a cell population in a sample obtained from a subject, and a programmed microprocessor. The programmed microprocessor is configured to run a method according to any one of the above claims.

In certain embodiments of any aspect of the invention, the kidney disease is transplant rejection. In certain embodiments of any aspect of the invention, the kidney disease is acute cellular rejection or acute humoral rejection. In certain embodiments of any aspect of the invention, the kidney disease is acute cellular rejection. In certain embodiments of any aspect of the invention, the kidney disease is acute humoral rejection.

### Examples

### 1. Methods

### Sample collection

Urine samples from patients having undergone kidney transplantation were collected by catheter or spontaneously. Samples were kept in a sterilized beaker and prepared for analysis within 6 hours of collection.

### Cell isolation

Samples were distributed in 50 ml tubes and centrifuged for 8 minutes at 4 °C and 1300 g. The supernatant was discarded and the pellets were resuspended and combined in PBS/BSA buffer, yielding 40 ml per sample. Analysis of non-fixed cells (T cells) was carried out on the day of sample collection. Analysis of fixed cells was carried out within 7 days of sample collection.

Fixed cells: For staining of intracellular markers, 10 ml of the isolated cells were transferred in a 15 ml tube and centrifuged for 8 minutes at 4 °C and 1300 g. The supernatant was discarded and the pellet was resuspended in PBS/BSA buffer. The solution was transferred to a 1.5 ml tube and centrifuged for 8 minutes at 4 °C and 2300 g. The supernatant was discarded. The pellet was resuspended in 100 µl PBS/BSA buffer and 100 µl 4% PFA incubated for 15 minutes at room temperature. Next, 1 ml of PBS was added and the sample was centrifuged for 8 minutes at 4 °C and 2300 g. The supernatant was discarded and the pellet was resuspended in 20 µl PBSA-BSA-acid solution for overnight storage or subjected to perforation for intracellular staining or.

Non-fixed cells: The remaining 30 ml of the isolated cells were slowly pipetted on 15 ml of saccharose-epichlorhydrin-copolymer, allowing isolation of mononuclear cells by centrifugation (20 minutes at 20 °C and 2000 g). Mononuclear cells are visible as white cellular layer and can be transferred to a 15 ml tube using a pipette. The cells are then centrifuged for 8 minutes at 4 °C and 1300 g. The pellet is resuspended in 1800 µl PBS/BSA buffer, distributed in two 1.5 ml tubes and centrifuged for 8 minutes at 4 °C and 1300 g. Next, the cells were subjected to staining.

### Staining

The following extracellular markers were used: CD3, CD4, CD8, CD10, EPCAM and podocalyxin. CD3, CD4 and CD8 were used to identify T cells. Intracellular cytokeratin staining of fixed cells was used identify epithelial cells. Cytokeratin and CD10 were used to identify proximal tubular epithelial cells. Cytokeratin and EPCAM were used to identify distal tubular epithelial cells. Podocalyxin was used to identify podocytes. Podocalyxin is a sialoglycoprotein and the main component of the glycocalix (extracellular carbohydrates bound to proteins or lipids) of podocytes. 4',6-Diamidin-2-phenylindol (DAPI) was used to distinguish living cells from dead cells within the non-fixed cells. It was added to the sample immediately before flow cytometry. Antibodies against CD3, CD4, CD8, CD10, EPCAM, podocalyxin and cytokeratin were coupled to the fluorescent dyes Vio Green, FITC, PE, PerCP, PE-Vio770, Cy5 and APC-Vio770. The emission spectra of the dyes can be clearly distinguished, allowing a separation of markers. All dyes can be excited by the three lasers of the MACSQuant Analyzer 10 flow cytometer.

Before staining, all samples were resuspended in 90 µl PBS/BSA buffer (extracellular staining) or 90 µl saponin (intracellular staining) and 10 µl flebogamma. Flebogamma blocks unspecific Fc receptors. Subsequently, antibodies were added. Samples were incubated in the dark for 15 minutes at 4 °C. Samples were washed using 1 ml PBS/BSA buffer (extracellular staining) or saponin (intracellular staining) and centrifuged for 8 minutes at 4 °C and 2300 g. The pellets are resuspended in 180 µl (panel 1) or 120 µl (panel 2 and 3). Samples which are stained with the antibodies in panel 2 and 3 were filtered using a "Cell Strainer 70 µm Nylon" (Corning) before flow cytometry. Table 1 lists the antibodies used in the experiments.

**Table 1.**

| **Panel 1: T cells** | **Panel 2: Podocytes** | **Panel 3: Tubular Epithelial Cells** | **Channels** |
|---|---|---|---|
| **DAPI** | | | **V1-Vio Blue-DAPI** |
| | | **Cytokeratin** | **B1-FITC** |
| | | FITC | |
| | | Miltenyi | |
| | | 130-080-101 | |
| | | 1:10 =10 µl in 100 µl | |
| | | Epithelial Cells | |
| | **Podocalyxin-PE** | | **B2-PE** |
| | REA246 | | |
| | Miltenyi | | |
| | 130-101-410 | | |
| | 1:10 =10 µl in 100 µl | | |
| | Podocyles | | |
| **CD3 PerCP** | | | **B3-PerCP-PI** |
| Miltenyi | | | |
| 130-094-965 | | | |
| 1:10=10µl in 100 µl | | | |
| T Cells | | | |
| **CD4-PE-Vio770** | | **CD10-PE-Vio770** | **Panel 1** |
| Miltenyi | | Miltenyi | |
| 130-096-552 | | 130-100-421 | |
| 1:10=10µl in 100 µl | | 1:10=10µl in 100 µl | |
| T Cells | | Proximal Tubular Epithelial Cells | |
| **CD8-APC-Vio770** | | **EPCAM APC-VIO 770** | **R2-APC Vio 770** |
| Miltenyi | | Miltenyi | |
| 130-096-561 | | 130-101-161 | |
| 10 µl in 100 µl | | 1:10=10µl in 100 µl | |
| T Cells | | Distal Tubular Epithelial Cells | |

### Flow cytometry

Samples were analysed and quantified by flow cytometry. A MACSQuant Analyzer 10 flow cytometer was used. Fig. 1 shows a typical example of flow cytometric analysis of the samples.

### 2. Patient cohorts

A longitudinal and a transversal cohort of kidney transplant patients were analysed.

### Transversal cohort

The transversal cohort comprises 19 patients that have received a biopsy of their transplant after showing signs of impaired kidney function (increased creatinin levels and/or proteinuria). Depending on the result of the biopsy, the patients were assigned to the groups acute cellular rejection, acute humoral rejection, chronic humoral rejection or no rejection. The group is heterogeneous with regard to the time after kidney transplantation. Patients were analyzed within three days of the biopsy. Exclusion criteria were increased immune suppression therapy and macroscopic hematuria. The control group comprises 9 patients with stable transplant function who have not received a kidney biopsy. Table 2 lists the characteristics of the patients in the transversal cohort. Age is given as the median; age ranges are indicated in brackets.

**Table 2**

| | **acute cellular rejection** | **acute humoral rejection** | **chronic humoral rejection** | **no rejection** | **control** |
|---|---|---|---|---|---|
| **n** | 4 | 3 | 4 | 8 | 9 |
| **Sex** | 4M/0F | 2M/1F | 3M/1F | 6M/2F | 7M/2F |
| **Age** | 65 (49-79) | 72 (49-73) | 51 (27-74) | 58 (32-79) | 50 (39-83) |

### Longitudinal cohort

The longitudinal cohort comprises 23 patients immediately after kidney transplantation. Patients were divided in the groups "complicated" and "uncomplicated". The group "complicated" comprises several complications including delayed graft function, rejection and surgical complications. Table 3 lists the characteristics of the patients in the longitudinal cohort. Age is given as the median; age ranges are indicated in brackets.

**Table 3**

| | **fresh transplantation** |
|---|---|
| **n** | 24 |
| **Sex** | 17M/7F |
| **Age** | 51 (22-77) |

### 3. Results

### Transversal cohort

A combination of the parameters T cell concentration (Fig. 2A), podocyte to T cell ratio (Fig. 2B) and podocyte to proximal tubular epithelial cell ratio (Fig. 2C) allowed for a mapping of patients to the different groups. The podocyte to T cell ratio allowed for identification of the patients undergoing acute cellular rejection. The combination of podocyte to T cell ratio and T cell concentration allowed for identification of the patients undergoing acute cellular rejection with a sensitivity of 100% and a specificity of 100%. The podocyte to proximal tubular epithelial cell ratio allowed for identification of the patients undergoing acute humoral rejection. The combination of podocyte to proximal tubular epithelial cell ratio and T cell concentration allowed for identification of the patients undergoing acute humoral rejection with a sensitivity of 100% and a specificity of 96,2%.

### Longitudinal cohort

Initially, more immune and epithelial cells were detected in the samples of the "uncomplicated" group. With time, the relation changed and more CD4+ and CD8+ cells, macrophages, podocytes, proximal and distal tubular epithelial cells were detected in the samples of the "complicated" group (all p<0.05).

## Claims

1. A method of assigning to a patient a likelihood of having a kidney disease, particularly a kidney disease selected from the group comprising kidney transplant rejection, acute renal failure, acute renal failure due to autoimmune disease, lupus nephritis, ANCA associated glomerulonephritis and diabetic kidney disease, or a likelihood of undergoing kidney transplant rejection, comprising the steps of
a. providing a urine sample from said patient;
b. determining a concentration of T cells, a concentration of podocytes and a concentration of proximal tubular epithelial cells, and optionally a concentration of distal tubular epithelial cells in said urine sample.

2. The method according to claim 1, wherein
- said T cells are **characterized by** expression of at least one of CD3, CD4 and CD8,
- said podocytes are **characterized by** expression of at least one of podocalyxin, podocin, nephrin and WT1,
- said proximal tubular epithelial cells are **characterized by** expression of CD10,
- said distal tubular epithelial cells are **characterized by** expression of EPCAM,
and wherein step b is effected by contacting said cells with a set of ligands, wherein said set comprises members capable of binding to each of CD4, CD8 and CD10, one of podocalyxin, podocin, nephrin and WT1, and optionally to cytokeratin, CD3 and/or EPCAM, wherein each of said members is capable of specifically binding to one of CD3, CD4, CD8, podocalyxin, podocin, nephrin, WT1, CD10, cytokeratin and EPCAM.

3. The method according to any one of the above claims, wherein said concentration of T cells, said concentration of podocytes, said concentration of proximal tubular epithelial cells and optionally, said concentration of distal tubular epithelial cells are each determined by flow cytometry.

4. The method according to any one of the above claims, further comprising the steps of
c. determining a podocyte/T cell ratio and a podocyte/proximal tubular epithelial cell ratio from said concentration of T cells, said concentration of podocytes, and said concentration of proximal tubular epithelial cells determined in step b;
d. comparing said podocyte/T cell ratio and said podocyte/proximal tubular epithelial cell ratio determined in step c with a threshold;
e. assigning to said patient a high likelihood of having a kidney disease or of undergoing kidney transplant rejection
i. if said podocyte/T cell ratio is below a podocyte/T cell threshold or
ii. if said podocyte/proximal tubular epithelial cell ratio is above a podocyte/proximal tubular epithelial cell threshold.

5. The method according to claim 4, wherein a high likelihood of having a kidney disease or of undergoing kidney transplant rejection is assigned to said patient in instances where additionally said concentration of T cells is above a T cell threshold, particularly wherein
- said T cell threshold is 2000 - 500 T cells/100 ml urine, particularly 1500 - 550 T cells/100 ml urine, more particularly 1000 - 600 T cells/100 ml urine, even more particularly approximately 600 T cells/100 ml urine;
- said podocyte/T cell threshold is 0.01 - 0.3, particularly 0.02 - 0.2, more particularly 0.1 - 0.2, even more particularly approximately 0.2;
- said podocyte/proximal tubular epithelial cell threshold is 1 - 13, particularly 1.5 to 10, more particularly 2 - 5, even more particularly approximately 3.

6. The method according to claim 5, wherein a high likelihood of *acute cellular* transplant rejection is assigned to said patient if said concentration of T cells is above said T cell threshold and said podocyte/T cell ratio is below said podocyte/T cell threshold.

7. The method according to claim 6, wherein a high likelihood of *acute humoral* transplant rejection is assigned to said patient if said concentration of T cells is above said T cell threshold and said podocyte/proximal tubular epithelial cell ratio is above said podocyte/proximal tubular epithelial cell threshold.

8. The method according to any one of claims 1 to 7, wherein said concentration of T cells, said concentration of podocytes, said concentration of proximal tubular epithelial cells and/or said concentration of distal tubular epithelial cells determined in step b are each compared with a respective predetermined cell type threshold, and a high likelihood of having a kidney disease or of undergoing kidney transplant rejection is assigned to said patient if at least two, particularly at least three, more particularly at least four, even more particularly all of said concentrations are above said respective predetermined thresholds.

9. The method according to any one of claims 1 to 7, wherein a total concentration of CD8+ T cells, CD4+ cells, podocytes, proximal tubular epithelial cells and distal tubular epithelial cells is determined and a high likelihood of having a kidney disease or of undergoing kidney transplant rejection is assigned to said patient if said total concentration is above a collective cell concentration threshold.

10. The method according to any one of the above claims, wherein said sample is provided on day 4 after a kidney transplantation or later, particularly on day 5 after a kidney transplantation or later.

11. A method of assigning a kidney transplant patient to increased immune suppression therapy comprising the steps of
- determining a likelihood of transplant rejection in said patient according to the method of any one of the above claims;
- assigning said patient to increased immune suppression therapy if a high likelihood of transplant rejection is determined;
particularly wherein
a. if a high likelihood of acute cellular transplant rejection is determined, the patient is assigned to *therapy suitable for acute cellular transplant rejection,* in particular to a therapy selected from the group comprising treatment with high dose cortisone, immune suppression therapy with increased doses of immune suppression drugs, treatment with therapeutic antibodies against T cells, treatment with an anti-thymocyte globulin and treatment with other therapeutic antibodies including anti-TNF;
b. if a high likelihood of acute humoral transplant rejection is determined, the patient is assigned to *therapy suitable for acute humoral transplant rejection,* in particular to a therapy selected from the group comprising treatment with high dose cortisone, plasma exchange and treatment with therapeutic antibodies against B cells, including Rituximab.

12. A method of assigning a kidney transplant patient to a kidney biopsy comprising the steps of
- determining a likelihood of transplant rejection in said patient according to the method of any one of the above claims;
- assigning said patient to kidney biopsy if a high likelihood of transplant rejection is determined.

13. A kit for determining the likelihood of a patient having a kidney disease or undergoing kidney transplant rejection, comprising an anti-CD8 and/or an anti-CD4 antibody, an anti-CD10 antibody, an antibody selected from an anti-podocalyxin antibody, an anti-podocin antibody, an anti-nephrin antibody and an anti-WT1 antibody, and optionally an anti-EPCAM antibody and/or an anti-cytokeratin antibody, wherein said antibodies are suitable for fluorescence based flow cytometry.

14. Use of a combination of antibodies for determining the likelihood of a patient having a kidney disease or undergoing kidney transplant rejection, wherein said combination of antibodies comprises an anti-CD8 and/or an anti-CD4 antibody, an anti-CD10 antibody, an antibody selected from an anti-podocalyxin antibody, an anti- podocin antibody, an anti-nephrin antibody and an anti-WT1 antibody, and optionally an anti-EPCAM antibody and/or an anti-cytokeratin antibody.

15. A system for determining the likelihood of a patient having a kidney disease or undergoing kidney transplant rejection, comprising
a. a device for determining the frequency of a cell population in a sample obtained from a subject, and
b. a programmed microprocessor,
wherein said programmed microprocessor is configured to run a method according to any one of the above claims.
